# EUROPEAN PATENT SPECIFICATION

(11) **EP 1 917 254 B1**
(45) Date of publication and mention of the grant of the patent: **17.12.2008**
(21) Application number: 06809226.1
(22) Date of filing: 27.07.2006
(51) Int. Cl.: C07D 295/02

(54) **PROCESS OF PRODUCING AMOROLFINE**
VERFAHREN ZUR HERSTELLUNG VON AMOROLFIN
PROCEDE DE PRODUCTION D'AMOROLFINE

(30) Priority: 28.07.2005 EP 05291612
(43) Date of publication of application: 07.05.2008
(73) Proprietor: Galderma S.A., 6330 Cham (CH)
(72) Inventor: WEBER, Beat, CH-4800 Zofingen (CH); ROSENBERGER, Stefan, CH-4800 Zofingen (CH)
(74) Representative: Allab, Myriam
(86) International application number: PCT/IB2006/003210
(87) International publication number: WO 2007/012983

(56) References cited:
- EP-A- 0 024 334
- FR-A- 2 589 858
- US-A- 4 384 116
- B. S. FRIEDMAN, F. L. MORRITZ: "The Alkylation of Benzene with Isoamylenes and with t-Pentyl Chloride" J. AM. CHEM. SOC, vol. 78, 1956, pages 2000-2002, XP002350771

## Description

The present invention relates to an improved process of producing Amorolfine base, which is an intermediate used in the production of Amorolfine (AMF) hydrochloride (Amorolfine HCl).

Amorolfine HCl is an active pharmaceutical ingredient (API) used in topical antimycotic (antifungal) compositions.

French Patent Application No 2,463,767 describes methods of producing Amorolfine HCl and intermediates in such production. In particular a method for the production of Amorolfine base (AMF base), which is a compound of formula (I): 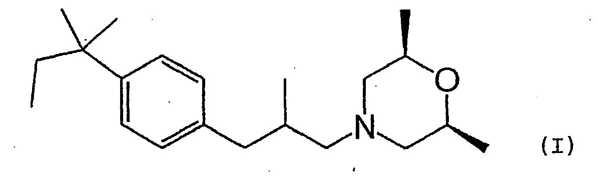 is described, the method involving the step of reacting a compound of the formula (a): 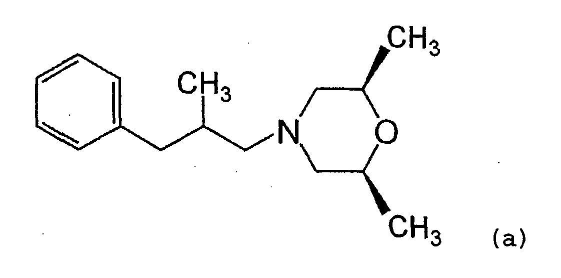 with a compound of the formula (b): 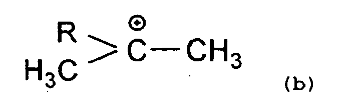 in a Friedel-Crafts alkylation to form AMF base. The suggested catalysts are those known for use as Friedel-Crafts catalysts, such as aluminium chloride, iron chloride, lead chloride, zinc chloride, boron trifluoride, hydrogen fluoride, sulphuric acid, and phosphoric acid. Sulfuric acid is stated to be the preferred catalyst. To furnish a compound of the formula (b):

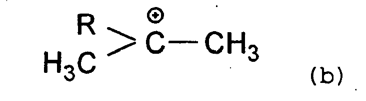

FR 2,463,767 suggests using tertiary alcohols such as 2-methyl-2-butanol, or tertiary chlorides such as 2-chloro-2-methylbutane. However, only 2-methyl-2-butanol is exemplified. The reaction temperature is noted as not being of critical importance but is suggested to be, in general, between 0 and 50 °C, preferably between 18 and 20 °C.

US patent application N° 4384116 discloses Friedel-Crafts alkylation of compound of the formula (a): 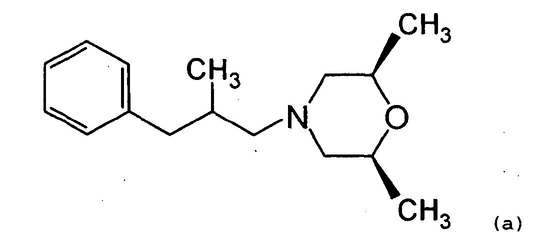 and derivatives thereof. Preferably, sulphuric acid and tertiary alcohols or olefins in a chlorinated hydrocarbon solvent are used. The reaction temperatures employed range from 0° to 50° C but are not critical.

There remains a need for improved processes for the production of Amorolfine salts, for example Amorolfine HCl, and its intermediates, such as Amorolfine base.

The inventors of the present invention have discovered that significant benefits can be obtained if a tertiary chloride such as 2-chloro-2-methylbutane is added to a compound of the formula (a): 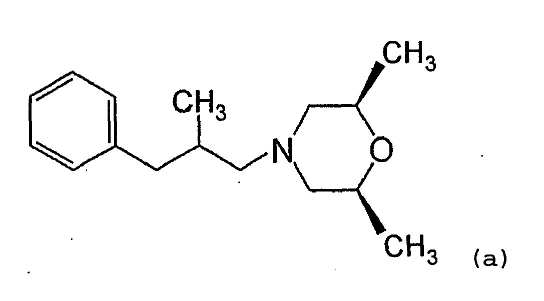 in mixture with FeCl3 as Friedel-Craft catalyst at a temperature between -40 to -60 °C, preferably around -50 °C.

The present invention provides an improved method of producing Amorolfine base, with a higher yield and lower impurity assay resulting.

According to a first aspect of the invention, there is provided a process of manufacturing a compound of formula (I): 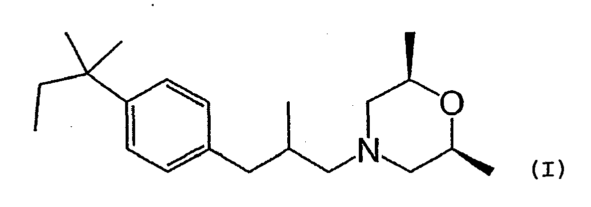 said process comprising the steps of:
(i) contacting a compound of formula (II): 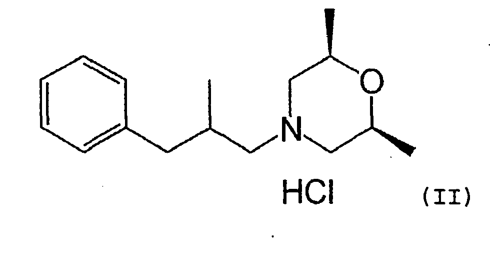 with FeCl3 as Friedel-Crafts catalyst at a temperature in the range of 20 to 30 °C; and
(ii) adding one equivalent of 2-halogeno-2-methylbutane,
characterised in that the reaction mixture obtained in step (i) is cooled to a temperature between -40 to -60 °C prior to step (ii) (addition of the 2-halogeno-2-methylbutane).

As used herein the term "Amorolfine base" (AMF base) refers to compounds of formula (I) and the term "bepromoline HCl" refers to compounds of formula (II).

As used herein the term "2-halogeno-2-methylbutane" refers to 2-methylbutane substituted in position 2 by an halogen atom chosen from the group consisting of bromine, chlorine, iodine, fluorine.

More preferably, the halogen is chlorine and consequently the 2-halogeno-2-methylbutane is 2-chloro-2-methylbutane.

The reaction mixture obtained in step (i) may typically be cooled to a temperature between -40 to -60 °C, generally -50 °C, prior to step (ii), i.e. addition of the 2-halogeno-2-methylbutane.

Friedel-Crafts catalyst FeCl₃ is generally used in dichloromethane (DCM).

Moreover the compound of formula (II) is generally present in 1 part of 2-halogeno-2-methylbutane per 1 part compound of formula (II).

In one embodiment, the process of producing a compound of formula (I) includes, after steps (i) and (ii) above, one or more of the following steps:
(a) pouring the reaction mixture from step (ii) onto an ice-water-mixture;
(b) separating the organic phase (i.e. DCM);
(c) washing the organic phase with optionally acidified, water,
(d) washing the organic phase with water;
(e) washing the organic phase from step (d) with a solution of sodium phosphate;
(f) washing the organic phase from step (e) with a solution of sodium hydroxide;
(g) washing the organic phase from step (f) with water;
(h) exchanging the dichloromethane solvent to toluene;
(i) performing toluene/water extractions;
(j) removing the toluene by distillation; and
(k) distilling the crude Amorolfine base from step (j).

As the preferred 2-halogeno-2-methylbutane is 2-chloro-2-methylbutane according to present invention, there is provided the preferred process of producing a compound of formula (I): 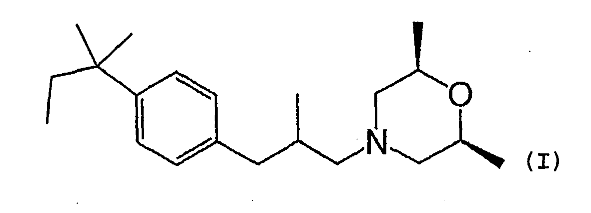 said process comprising the steps of:
(i) contacting a compound of formula (II): 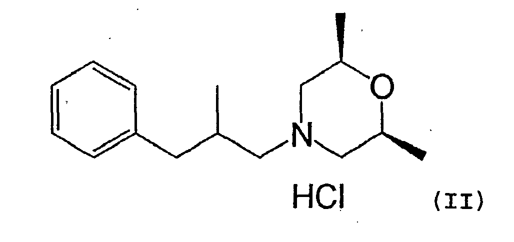 with FeCl3 as Friedel-Crafts catalyst at a temperature in the range of 20 to 30 °C; and
(ii) adding 2-chloro-2-methylbutane, characterised in that the reaction mixture obtained in step (i) is cooled to a temperature between -40 to -60 °C prior to step (ii) (addition of the 2-chloro-2-methylbutane).

This process can be performed as described above and can comprise one or more of the steps (a) to (k) above.

According to the present invention, the process of producing a compound of formula (I): 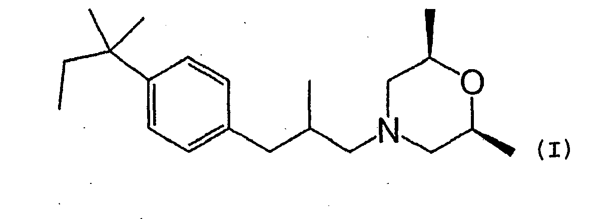 comprises steps (i) and (ii). Said steps can be preceded by the step of contacting a compound of the formula (III): 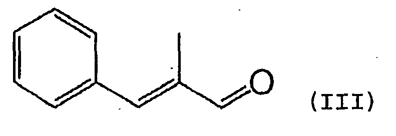 with a compound of formula (IV): 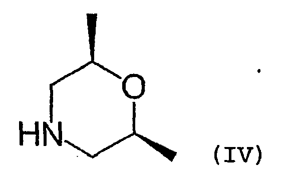 in the presence of a catalyst such as palladium precipitated onto carbon, methanol and hydrogen gas, wherein the step of contacting the compound of the formula (III) with the compound of formula (IV) is optionally conducted under basic conditions, with acetic acid added once the consumption of the hydrogen gas has ceased.

Compounds of formulae (III) and (IV) are termed herein "α-methylcinnamaldehyde" and "cis-2,6-dimethyl morpholine" (DMM), respectively.

The basic conditions are generally provided by KOH, typically, 1.8 mol-% KOH.

According to a second aspect of the present invention, there is provided a process of producing a compound of formula (V): 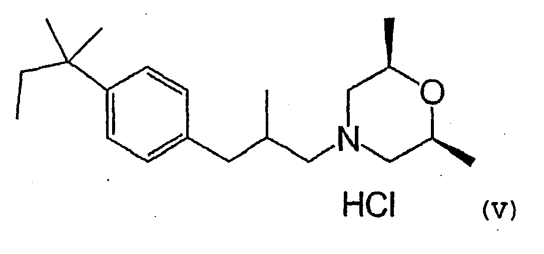 comprising a process as described above for the first aspect of the present invention.

This compound of formula (V) can be obtained from Amorolfine base (compound (I)) thanks to a salification step.

Typical and usual features of each aspect of the invention are as for each of the other aspects of the invention *mutatis mutandis.*

Throughout the specification, unless the context demands otherwise, the terms "comprise" or "include", or variations such as "comprising" and "including" will be understood to imply the inclusion of a stated feature, or group of features, but not to the exclusion of any other feature, or group of features.

The present invention will now be described by way of example only, with reference to the following examples which are not intended to be limiting on the invention.

### Example 1: Production of Bepromoline HCl

### a) General considerations:

A mixture of 1 part of α-methyl-cinnamaldehyde to one part of cis-2,6-dimethyl-morpholine (DMM) is hydrogenated in methanol in the presence of catalytic amount of palladium on carbon optionally under basic conditions until the up-take of H₂ gas ceases, this indicating completion of the reduction of the C=C double bond. Acetic acid is then added for the reduction of the C=N double bond under hydrogen pressure; the C=N double bond is formed between the aldehyde and the amino moiety of the two reactants, α-methyl-cinnamaldehyde and DMM, respectively.

The catalyst is then filtered off and the methanol is removed by distillation. Toluene is added and the inorganic components are removed by washing with water. Toluene and unreacted DMM are distilled off. Then fresh toluene is added and HCl gas is bubbled through the solution. The pH is adjusted to 3-4. The bepromoline HCl is centrifuged and dried.

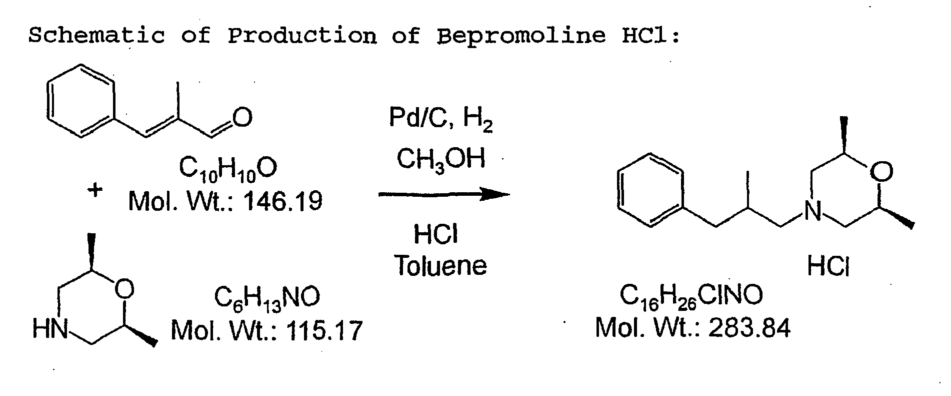

### Provision of Basic Conditions

Basic conditions were provided by KOH, which is used to neutralise the acidic components present in the α-methyl-cinnamaldehyde. The absence of traces of acid improved the kinetic of the reaction. The reduction of the aldehyde function to the corresponding alcohol is avoided by addition of KOH.

### Solvent

Methanol might be substituted by toluene to avoid the later solvent exchange step.

### Temperature of Hydrogenation

40 °C is the optimum temperature for both hydrogenation steps. However, the temperature may typically be set at no more than 45 °C, preferably between 30 and 45 °C.

### Acetic Acid

The reduction of the C=N double bond formed between the aldehyde and the amino function of the two components is conducted under hydrogen pressure in acidic conditions after the addition of acetic acid.

A molar ratio of acetic acid to KOH is around 1.3 (±10%).

The acetic acid is typically added at a temperature range of between 40 to 45 °C, and no more than 45 °C.

### Toluene Exchange

The toluene is advantageously added to facilitate the phase separations and the distillation step of the un-reacted DMM, thus improving the purity of bepromoline.

### Bepromoline.HCl purity

The trans isomers (VI) and (VII) of bepromoline, coming from trans isomers presents as by products in the 2,6-dimethyl morpholine starting material, are partially eliminated during the crystallization of bepromoline HCl.

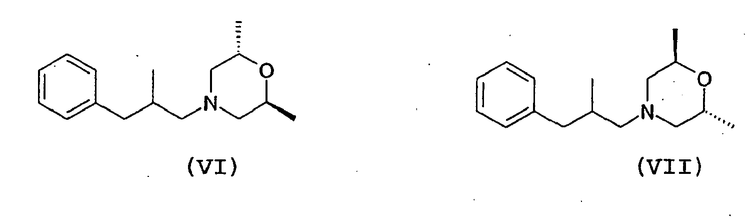

The purity of the bepromoline HCl (cis isomer) is superior or equal to 99,5%.

### Stability Temperature

The product is stable up to 150 °C.

### b) Synthesis:

### (Weights are given for 1 kmol α-methylcinnamaldehyde).

A reactor was charged with 146 kg α-methylcinnamaldehyde, 115 kg cis-2,6-dimethyl-morpholine, 2.1 kg 50% KOH, 278 kg methanol and 5.8 kg of a palladium/carbon catalyst and then filled with hydrogen at 15-25 °C.

The hydrogenation was then run at a pressure of ∼2 bar and 35-45 °C until H₂ consumption ceased.

1.5 kg acetic acid was then added, and the hydrogenation was re-commenced. The hydrogenation was conducted at a pressure of ∼2 bar and at a temperature of 40-45 °C until no further H₂ was consumed.

The reaction mixture was filtered and the catalyst washed with methanol and purified water The solvents were distilled of at a temperature of up to 95 °C under vacuum.

Two extractions were performed using toluene and water. The waste water was drained off.

The solvent was then distilled off under vacuum.

The reactor was charged with 904 kg toluene and 33 kg HCl gas at a temperature of up to 50 °C. Then the pH was adjusted to 3 - 4. The reaction mixture was cooled and then stored under agitation sufficiently to reached complete cristallization.

The mixture was centrifuged and washed with cold (0-5 °C) toluene. A second crop of Bepromoline HCl was isolated from the mother liquor.

The process yielded 287 kg wet bepromoline HCl, which was then dried at 60 °C under vacuum. After drying, the first crop of Bepromoline HCl was 227kg and the second 18kg. This corresponds to a yield of 87%. (80% for the first crop Bepromoline HCl and 7% for the second crop)

### Example 2: Production of Amorolfine Base

### a) General considerations:

1 part bepromoline.HCl is treated with 1.3 parts FeCl₃ ±5% in dichloromethane at room temperature. The resulting slurry is cooled to approximately - 50°C, whereupon 1 to 1.1 parts of 2-chloro-2-methylbutane is added.

After an appropriate reaction time of around 2.5 hours, the reaction mixture is poured onto an ice-water mixture. The organic phase is separated and washed with acidic water, and then with sodium phosphate solution and with sodium hydroxide solution. After a stripping with toluene, extractions with water are performed. The solvent is then removed. Then the residue is distilled.

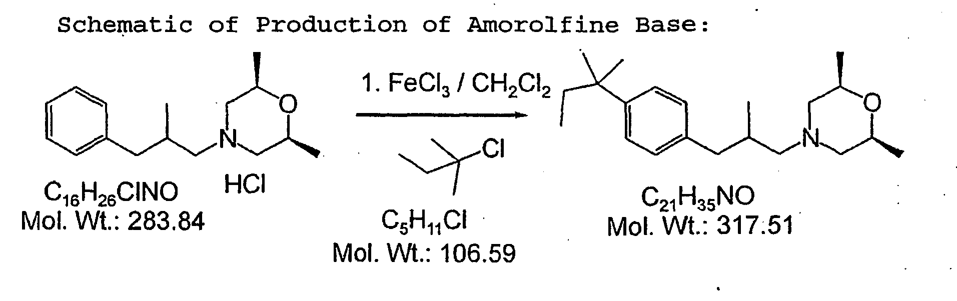

### Reaction Temperature for the addition of FeCl3 to Bepromoline HCl

The addition of FeCl3 to Bepromoline HCl takes place at room temperature. At lower temperatures the subsequent Friedel-Crafts alkylation fails partially or completely (Table 1)

**Table 1**

| Temperature (°C) | Bepromoline assay in the crude Amorolfine base (%) |
|---|---|
| 20-30 | 8-14 |
| 0 | 14 |
| -20 | 100 |

### Friedel-Crafts Catalysts

A suitable molar ratio of FeCl₃ to bepromoline is 1:2 to 1:5 equivalents of catalyst. 1.3 equivalents of FeCl₃ is preferred.

### Reaction Temperature for Friedel-Crafts Alkylation

To decrease the fenpropimorph (FPM) by-product, the reaction is conducted at low temperature, preferably -50°C (see Table 2):

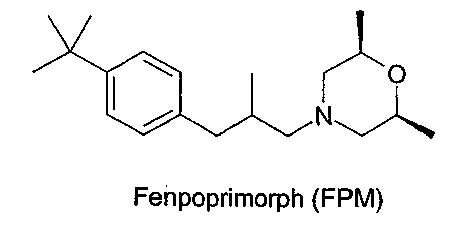

**Table 2**

| Temperature (°C) | FPM (%) |
|---|---|
| -52 to -49 | 0.14-0.25 |
| -40 | 1.7 |
| -35 | 2.0 |
| -20 | 2.7 |

Fenpropimorph (FPM) is a problematic by-product as it is difficult to remove from the end product.

### Ratio of Bepromoline HCl to 2-chloro-2-methylbutane

Batches were performed with 10 % excess 2-chloro-2-methylbutane and at a 1:1 ratio. The FPM assay is lower for the 1:1 ratio and thus this proportion is preferred.

### Phosphate and Alkaline Extraction

The Amorolfine HCl (which is in the DCM) is converted to the free base during these extractions. Phosphate was used to remove traces of Fe.

### Solvent Exchange

Advantages result if the solvents are exchanged (i.e. toluene in place of DCM): the volume is reduced and the waste-water is contaminated with less chlorinated solvent.

### Toluene-water Extraction

Those extractions are necessary to get the appropriate quality for the subsequent distillation. If these extractions are omitted, the Amorolfine base slightly decomposes at 180°C. The distillation become very sluggish and fumes are formed. The vacuum distillation is then not possible at plant scale.

The yield was approximately 90% of crude Amorolfine base.

### b) Synthesis:

### (weights are given for 1 kmol bepromoline HCl)

The reactor was charged with 212 kg FeCl₃ and 757 kg DCM. 284 kg bepromoline HCl in 946 kg DCM were added to the reactor at 20-30 °C. The reaction mixture was completed with 213 kg DCM and cooled to -50°C. 107 kg 2-chloro-2-methylbutane in 107 kg DCM were added at -50 °C, although a temperature of -60 to -45 °C is acceptable, and stirred for 2.5 hours. Hydrolysis was performed using 255 kg ice and 785 kg water.

Phase separation was then performed.

Extractions using slightly acidic water (water and diluted HCl) were performed, followed by a further extraction with a solution of Na₃PO₄ in water. A subsequent extraction was conducted using NaOH diluted in water to a pH ≥13. At a lower pH value there is incomplete HCl removal, leading to distillation problems. Two washes were performed with water.

The solvent was distilled off.

Toluene was added and four water extractions were performed. Finally the solvent was distilled off under vacuum.

This yielded 283 kg crude AMF (approximately 90% AMF base crude).

### Example 3: Distillation of Amorolfine Base

### a) General considerations:

The distillation step is necessary to purify Amorolfine Base.

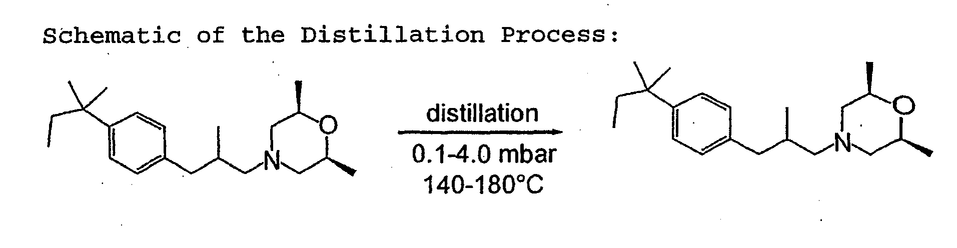

### b) Distillation:

283 kg of crude Amorolfine base are distilled at 141°-144°C under reduced pressure (typically 0.14-0.15 mbar). The fractions are combined in such a way that the impurity profile of the combined material is within the desired specification.

After distillation, 190 kg AMF base were produced (approximately 67% AMF base distilled).

### Example 4: Production of Amorolfine HCl and evaluation of the purity of the produced compound

### a) General considerations:

i) purpose: The aim of this stage is to ensure that sufficient impurities are properly removed with the formation of the Amorolfine HCl and only one crystallisation step with ethanol being used.
ii) production of Amorolfine HCl with Amorolfine base (salification step): HCl gas is added to a solution of Amorolfine base in two parts of ethanol until the pH reaches 1.5 to 3. The Amorolfine HCl crystallises at around 45°C. The slurry is cooled to no less than -15°C (which should take no less than 2 hours). The crude Amorolfine HCl is isolated by centrifugation and washed with cold ethanol. The crude Amorolfine HCl is then re-crystallised at between -20 to -15 °C from two parts of ethanol.

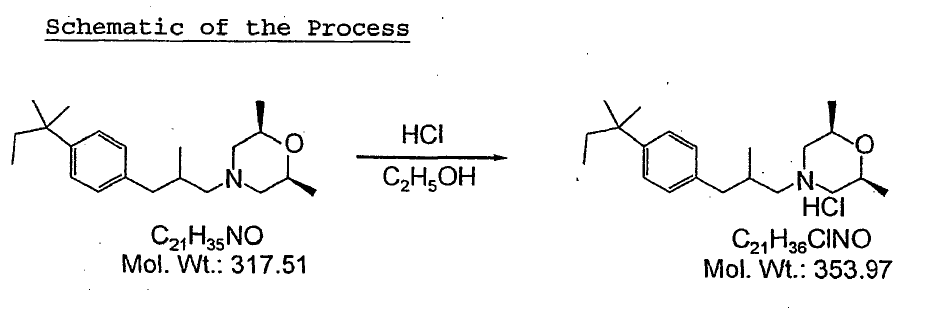

### Amounts of by-products in the Amorolfine base

Apart from FPM, all impurities present in AMF base are removed firstly by the salification of AMF base into AMF HCl and secondly by one crystallization step from ethanol.

The data given in Table 3 were taken from different crystallizations experiments.

**Table 3**

| | Bepromoline (%) | FPM (%) | Trans-isomers (%) |
|---|---|---|---|
| AMF base | 5 | 0.25 | 0.5 |
| AMF HCl crude | 0.3 | 0.25 | 0.3 |
| AMF HCl | <0.1 | 0.25 | <0.2 |
| Required spec. | <0.2 | <0.3 | <0.2 |

### Reaction Temperature

During the addition of the HCl gas, the temperature raises by around 35°C. This exotherm is used to warm the batch. After the addition of HCl the temperature is raised to a level that ensures that the reaction mixture is in solution.

The final temperature of -20 to -15°C is important to obtain an optimum yield.

### Re-crystallisation of the Amorolfine HCl

Ethanol is the preferred solvent. The Amorolfine HCl is dissolved in hot ethanol and this solution is filtered to remove foreign matter. The filtrate is then cooled to -15 to -20°C to get the optimum yield for cristallization. After centrifugation, the crystals are washed with an appropriate amount of ethanol.

### Drying

The Amorolfine HCl is stable up to 150°C. Drying conditions of 60°C in a vacuum are used and do not produce any problems with the residual solvent.

### b) Synthesis:

### (Weights are given for 1kmol AMF base)

The reactor was charged with 317 kg AMF and 640 kg ethanol. 38 kg HCl gas was added at 10-65 °C. The reaction mixture was then heated to 60 °C, followed by cooling to -15 to -20 °C. The mixture was stored for 30 minutes to 2 hours.

The Amorolfine HCl was centrifuged and washed with 210 kg of ethanol.

2 parts ethanol were used to dissolve the Amorolfine HCl at 70-80 °C.

The hot solution was filtered and the filter rinced with 15 kg hot ethanol. The filtrate was then cooled to -15 to -20 °C and stored for 30 minutes to 2 hours.

The crystallized Amorolfine HCl was centrifuged and washed with 210 kg of ethanol.

The mixture was then dried at a temperature of 60 °C under vacuum (<100 mbar).

This yielded 271 kg AMF HCl. The yield was approximately 77%

## Claims

1. Process of manufacturing a compound of formula (I) : 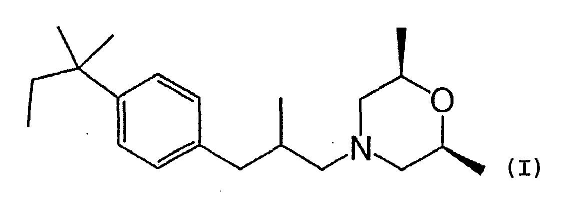 said process comprising the steps of:
(i) contacting a compound of formula (II): 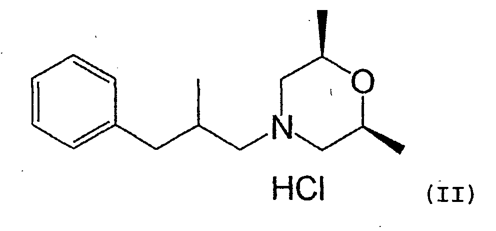 with FeCl3 as Friedel-Crafts catalyst at a temperature in the range of 20 to 30 °C; and
(ii) adding one equivalent of 2-halogeno-2-methylbutane,
**characterised in that** the reaction mixture obtained in step (i) is cooled to a temperature between -40 to -60 °C prior to step (ii).

2. Process as claimed in claim 1 wherein the reaction mixture obtained in step (i) is cooled to a temperature of -50 °C prior to step (ii).

3. Process as claimed in any preceding claim wherein the Friedel-Crafts catalyst FeCl3 is used in dichloromethane.

4. Process as claimed in any preceding claim, wherein the compound of formula (II) is present in 1 part of 2-halogeno-2-methylbutane per 1 part of the compound of formula (II).

5. Process as claimed in any preceding claim wherein said process further includes one or more of the following steps:
(a) pouring the reaction mixture from step (ii) onto an ice-water mixture;
(b) separating the organic phase (i.e. DCM);
(c) washing the organic phase with optionally acidified, water,
(d) washing the organic phase with water;
(e) washing the organic phase from step (d) with a solution of sodium phosphate;
(f) washing the organic phase from step (e) with a solution of sodium hydroxide;
(g) washing the organic phase from step (f) with water;
(h) exchanging the dichloromethane solvent to toluene;
(i) performing toluene/water extractions;
(j) removing the toluene by distillation; and
(k) distilling the crude Amorolfine base from step (j).

6. Process as claimed in any preceding claim, wherein said 2-halogeno-2-methylbutane is 2-chloro-2-methylbutane.

7. Process of producing a compound of formula (V): 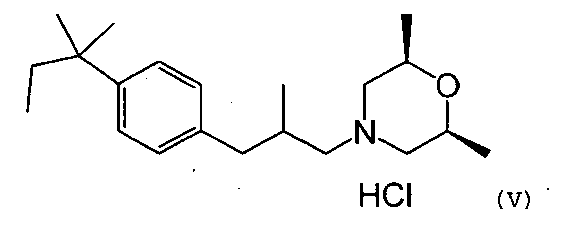 comprising a process as claimed in any preceding claim in order to obtain the compound of formula (I), wherein said process further includes the salification of the compound of formula (I).

## Patentansprüche

1. Verfahren zur Herstellung einer Verbindung der Formel (I): 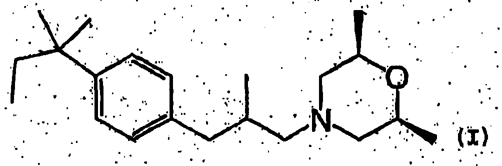 wobei das Verfahren die folgenden Schritte umfasst:
(i) Inkontaktbringen einer Verbindung der Formel (II): 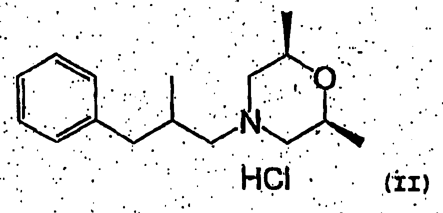 mit FeCl₃ als Friedel-Crafts-Katalysator bei einer Temperatur im Bereich von 20 bis 30 °C und
(ii) Zugabe eines Äquivalents 2-Halogen-2-methylbutan, **dadurch gekennzeichnet, dass** das in Schritt (i) erhaltene Reaktionsgemisch vor dem Schritt (ii) auf eine Temperatur von -40 bis -60 °C abgekühlt wird.

2. Verfahren nach Anspruch 1, wobei das in Schritt (i) erhaltene Reaktionsgemisch vor Schritt (ii) auf eine Temperatur von -50 °C abgekühlt wird.

3. Verfahren nach einem der vorhergehenden Ansprüche, wobei der Friedel-Crafts-Katalysator FeCl₃ in Dichlormethan verwendet wird.

4. Verfahren nach einem der vorhergehenden Ansprüche, wobei die Verbindung der Formel (II) in 1 Teil 2-Halogen-2-methylbutan auf 1 Teil der Verbindung der Formel (II) vorliegt.

5. Verfahren nach einem der vorhergehenden Ansprüche, wobei das Verfahren ferner einen oder mehrere der folgenden Schritte umfasst:
(a) das Reaktionsgemisch aus Schritt (ii) wird auf ein EisWasser-Gemisch gegossen;
(b) die organische Phase (d. h. DCM) wird abgetrennt;
(c) die organische Phase wird mit gegebenenfalls angesäuertem Wasser gewaschen;
(d) die organische Phase wird mit Wasser gewaschen;
(e) die organische Phase aus Schritt (d) wird mit einer Natriumphosphat-Lösung gewaschen;
(f) die organische Phase aus Schritt (e) wird mit einer Natriumhydroxid-Lösung gewaschen;
(g) die organische Phase aus Schritt (f) wird mit Wasser gewaschen;
(h) das Lösungsmittel Dichlormethan wird gegen Toluol ausgetauscht;
(i) es werden Toluol/Wasser-Extraktionen durchgeführt;
(j) das Toluol wird durch Destillieren entfernt; und
(k) die rohe Amorolfin-Base aus Schritt (j) wird destilliert.

6. Verfahren nach einem der vorhergehenden Ansprüche, wobei das 2-Halogen-2-methylbutan das 2-Chlor-2-methylbutan ist.

7. Verfahren zur Herstellung einer Verbindung der Formel (V): 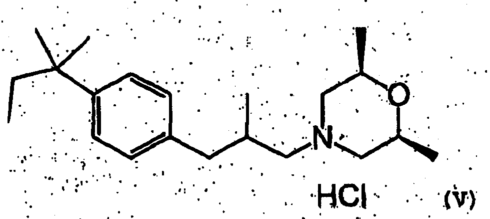 das das Verfahren nach einem der vorhergehenden Ansprüche umfasst, um die Verbindung der Formel (I) herzustellen, wobei das Verfahren ferner die Salzbildung der Verbindung der Formel (I) umfasst.

## Revendications

1. Procédé de fabrication d'un composé de formule (I) : 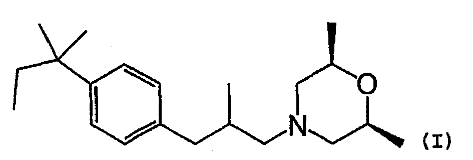 ledit procédé comprenant les étapes consistant à :
(i) mettre en contact un composé de formule (II) : 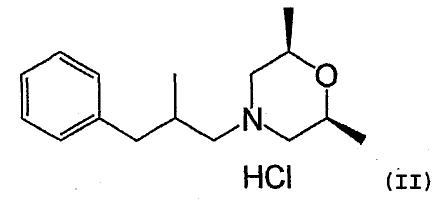 avec du FeCl₃ comme catalyseur de Friedel-Crafts à une température dans la plage allant de 20 à 30°C ; et
(ii) ajouter un équivalent de 2-halogéno-2-méthylbutane,
**caractérisé en ce que** le mélange réactionnel obtenu dans l'étape (i) est refroidi jusqu'à une température allant de -40 à -60°C préalablement à l'étape (ii).

2. Procédé selon la revendication 1, dans lequel le mélange réactionnel obtenu dans l'étape (i) est refroidi jusqu'à une température de -50°C préalablement à l'étape (ii).

3. Procédé selon l'une quelconque des revendications précédentes, dans lequel le catalyseur de Friedel-Crafts, FeCl₃, est utilisé dans du dichlorométhane.

4. Procédé selon l'une quelconque des revendications précédentes, dans lequel le composé de formule (II) est présent selon 1 partie de 2-halogéno-2-méthylbutane pour 1 partie du composé de formule (II).

5. Procédé selon l'une quelconque des revendications précédentes, dans lequel ledit procédé comporte en outre une ou plusieurs parmi les étapes suivantes consistant à :
(a) verser le mélange réactionnel de l'étape (ii) dans un mélange d'eau glacée ;
(b) séparer la phase organique (c'est-à-dire le DCM) ;
(c) laver la phase organique par de l'eau éventuellement acidifiée ;
(d) laver la phase organique par de l'eau ;
(e) laver la phase organique de l'étape (d) par une solution de phosphate de sodium ;
(f) laver la phase organique de l'étape (e) par une solution d'hydroxyde de sodium ;
(g) laver la phase organique de l'étape (f) par de l'eau ;
(h) échanger le solvant de dichlorométhane pour du toluène ;
(i) réaliser des extractions toluène/eau ;
(j) éliminer le toluène par distillation ; et
(k) distiller la base d'Amorolfine brute issue de l'étape (j).

6. Procédé selon l'une quelconque des revendications précédentes, dans lequel ledit 2-halogéno-2-méthylbutane est le 2-chloro-2-méthylbutane.

7. Procédé de production d'un composé de formule (V) : 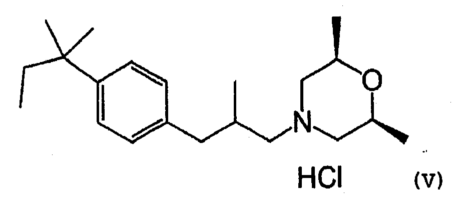 comprenant un procédé selon l'une quelconque des revendications précédentes, afin d'obtenir le composé de formule (I), dans lequel ledit procédé comporte en outre la salification du composé de formule (I).
